Europäisches Patentamt

European Patent Office

Office européen des brevets

(11) Numéro de publication: **0 143 046**

**A2**

(12) **DEMANDE DE BREVET EUROPEEN**

(21) Numéro de dépôt: **84402283.0**

(22) Date de dépôt: **12.11.84**

(51) Int. Cl.⁴: **H 01 L 41/08**
**A 61 B 5/08, H 04 R 17/00**
**G 01 L 1/16**

(30) Priorité: **10.11.83 FR 8317963**

(43) Date de publication de la demande:
**29.05.85 Bulletin 85/22**

(84) Etats contractants désignés:
**DE FR GB**

(71) Demandeur: **Lewiner, Jacques**
**5, rue Bory d'Arnex**
**F-92210 Saint-Cloud(FR)**

(71) Demandeur: **Hennion, Claude**
**18, rue Flatters**
**F-75005 Paris(FR)**

(72) Inventeur: **Lewiner, Jacques**
**5, rue Bory d'Arnex**
**F-92210 Saint-Cloud(FR)**

(72) Inventeur: **Hennion, Claude**
**18, rue Flatters**
**F-75005 Paris(FR)**

(74) Mandataire: **Behaghel, Pierre**
**CABINET PLASSERAUD 84 rue d'Amsterdam**
**F-75009 Paris(FR)**

(54) Perfectionnements aux nappes composites constitutives de transducteurs électroméchniques et aux transducteurs équipés de telles nappes.

(57) L'invention concerne une nappe composite souple destinée à constituer une alèse se comportant comme un transducteur électromécanique en vue de détecter les apnées de sujets couchés sur elle. Cette nappe comprend, en plus d'une feuille (1) sensible aux pressions au sens de la transduction électromécanique (électret ou feuille piézoélectrique polarisée), de deux électrodes minces (2,3), de feuilles de protection (5, 6) et de cales d'écartement (13) placées entre les deux feuilles contre lesquelles elles sont juxtaposées et entre lesquelles elles créent des chambres, des pastilles fixées sur les feuilles de protection, en regard des chambres.

Fig.2.

PERFECTIONNEMENTS AUX NAPPES COMPOSITES CONSTITUTIVES
DE TRANSDUCTEURS ELECTROMECANIQUES ET AUX TRANS-
DUCTEURS EQUIPES DE TELLES NAPPES

L'invention est relative aux nappes composites constitutives de transaucteurs électromécaniques et plus particulièrement à celles, de ces nappes, qui présentent une certaine souplesse et s'étendent sur une relativement grande surface, cette surface étant de préférence de l'ordre de plusieurs décimètres carrés.

Elle vise également les transducteurs équipés de telles nappes composites.

On sait que ces transducteurs permettent de transformer en variations d'une tension électrique les variations de pression qui sont exercées localement sur la nappe et/ou les déformations locales de cette nappe.

Les nappes composites en question comprennent essentiellement à cet effet une feuille diélectrique "sensible" à la pression au sens de la transduction électromécanique, feuille chargée électriquement en permanence ou porteuse d'une polarisation électrique permanente, interposée entre deux électrodes ou pellicules conductrices de l'électricité elles-mêmes recouvertes extérieurement de deux feuilles de protection, et les transducteurs équipés de ces nappes composites comportent, en plus de celles-ci, des moyens électriques d'exploitation et éventuellement d'alarme connectés aux deux électrodes desdites nappes composites.

Une application intéressante de ces transauc-teurs, à laquelle l'invention se rapporte de préférence, mais non exclusivement, est la détection des apnées, la surveillance des fonctions biologiques (respiration, rythme cardiaque,...), en particulier chez les nourrissons.

On constitue à cet effet, à l'aide des nappes composites considérées, des alèses que l'on interpose entre un matelas et le sujet à surveiller, couché, assis ou appuyé sur le matelas : les mouvements respiratoires ou cardiaques de ce sujet provoquent des variations locales cycliques de la pression exercée sur cette alèse, variations qui se traduisent par des variations cycliques semblables d'une tension électrique et les moyens d'exploitation sont agencés de façon à exciter l'alarme lorsque lesdites variations cycliques de tension cessent pendant une durée qui dépasse un seuil estimé dangereux ou présentent une forme s'écartant dans une mesure excessive d'une forme considérée comme normale.

L'invention est relative à celles des nappes composites ci-dessus décrites, dans lesquelles des cales d'écartement sont prévues entre la feuille sensible et l'une des feuilles de protection délimitant ainsi à l'intérieur de la nappe des chambres.

Elle s'applique à l'une et/ou à l'autre des dispositions suivantes :

- dans le cas où la feuille sensible est chargée électriquement en permanence et crée un champ électrique extérieur, les cales sont directement interposées entre cette feuille sensible et l'une des deux électrodes, ce qui forme des chambres délimitées par cette électrode, cette feuille sensible et ces cales,

- dans le cas où la feuille sensible est une feuille piézoélectrique polarisée électriquement en permanence et interposée entre les deux électrodes, les cales sont directement interposées entre l'une de ces électrodes et la feuille de protection extérieure correspondante, ce qui forme des chambres délimitées par cette électrode, cette feuille de protection et ces cales.

3                                   **0143046**

L'invention a pour but surtout, d'améliorer la sensibilité des transducteurs du genre en question.

A cet effet, les nappes composites du genre ci-dessus sont essentiellement caractérisées en ce qu'elles présentent des surépaisseurs localisées en regard des chambres.

Dans des modes de réalisation préférés, on a recours en outre à l'une et/ou à l'autre des dispositions suivantes :

- les surépaisseurs sont constituées par des reliefs faisant saillie sur la face extérieure de une au moins des feuilles de protection,

- les surépaisseurs ont un contour semblable à celui des chambres en regard desquelles elles sont placées mais plus petit,

- les surépaisseurs présentent une épaisseur sensiblement constante sur toute leur étendue,

- les surépaisseurs ont une épaisseur non uniforme, décroissante vers la périphérie,

- les surépaisseurs sont constituées dans un matériau de dureté au moins égale à celles des feuilles de protection,

- les surépaisseurs sont constituées en un matériau de compressibilité au moins égale à celle des feuilles de protection,

- les surépaisseurs sont constituées dans le même matériau que les feuilles de protection,

- les surépaisseurs sont rapportées par collage sur la feuille de protection,

- les surépaisseurs font partie intégrante des feuilles de protection,

- les surépaisseurs ont une épaisseur comprise entre 0,5 et 5 mm,

- les diverses couches de la nappe comprennent des moyens pour empêcher le déplacement au cours du temps des surépaisseurs par rapport aux chambres,

- la nappe composite constitue une alèse rectangulaire pour nourrissons dont l'un des côtés est compris entre 10 et 40 cm et l'autre côté entre 20 et 50 cm.

L'invention comprend, mises à part ces dispositions principales, certaines autres dispositions qui s'utilisent de préférence en même temps et dont il sera plus explicitement question ci-après.

Dans ce qui suit, l'on va décrire deux modes de réalisation préférés de l'invention en se référant aux dessins ci-annexés d'une manière bien entendu non limitative.

La figure 1, de ce dessin, montre en vue perspective, portions arrachées, une alèse établie conformément à l'invention.

La figure 2 montre à plus grand échelle, en coupe transversale, un morceau de cette alèse.

La figure 3 montre à encore plus grande échelle, en coupe transversale, un détail dudit morceau.

La figure 4 montre semblablement à la figure 2 une variante d'un morceau d'alèse également conforme à l'invention.

La figure 5 montre, en vue de profil, la partie d'un bras articulé de robot réalisée conformément à l'invention.

La figure 6, montre, en vue perspective, une partie d'un assemblage de capteurs sensibles à la pression également conforme à l'invention.

On fait comprendre à l'alèse A une feuille diélectrique 1 interposée entre deux feuilles, pellicules ou couches conductrices de l'électricité 2 et 3, désignées dans la suite par le mot "électrodes".

La feuille 1 est choisie de façon telle qu'elle soit "sensible" au sens de la transduction électromécanique, c'est-à-dire qu'elle soit capable de transformer les variations de pression appliquées localement sur elle en variations de la tension électrique recueillie aux bornes des deux électrodes 2 et 3.

A cet effet, elle est avantageusement constituée en une matière plastique telle que le polypropylène, qu'un P.T.F.E., qu'un poly(éthylène-propylène fluoré) ou encore qu'un polymère ou copolymère à base de fluorure de polyvinylidène, son épaisseur étant de préférence comprise entre 5 et 50 microns.

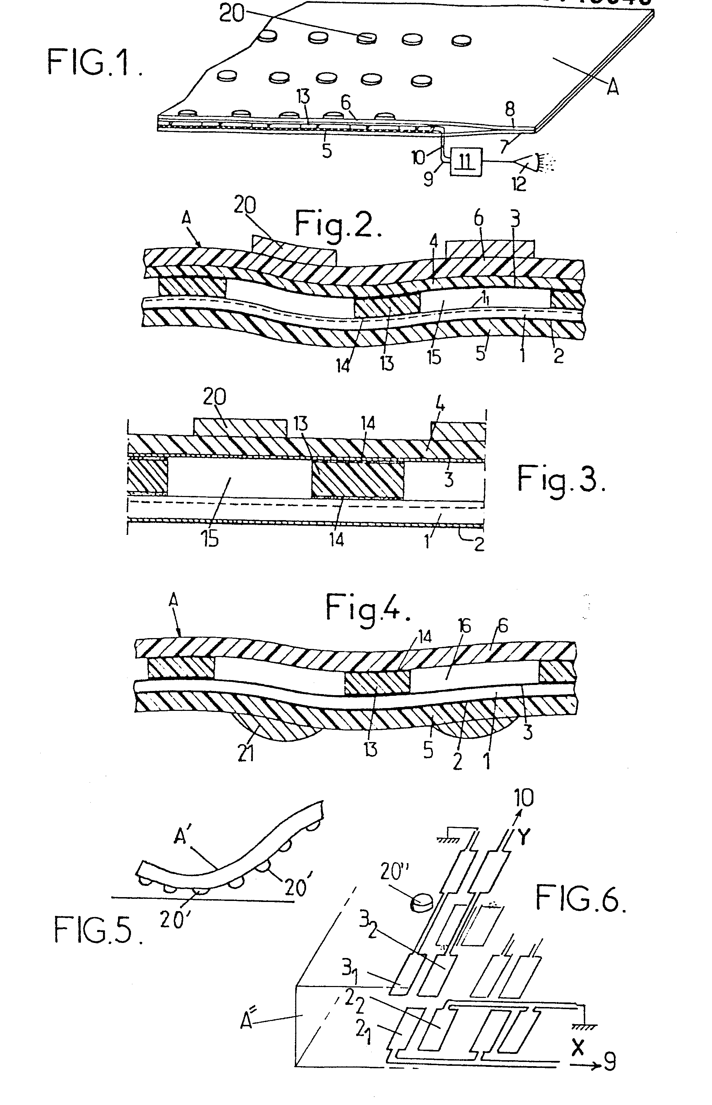

Cette feuille 1 est porteuse d'une polarisation électrique permanente ou chargée électriquement en permanence au moins au voisinage d'une $1_1$ de ses

faces, par des charges positives et/ou négatives qui ont été schématisées par le signe - sur la figure 2.

Dans ce dernier cas (charge électrique permanente), la feuille 1 crée un champ électrique extérieur et elle est maintenue écartée, au moins en partie, de l'une des électrodes 3 par des intervalles isolants, comme visible sur la figure 2.

Dans l'autre cas (polarisation électrique permanente), schématisé sur la figure 4, la feuille 1 possède des propriétés piézoélectriques et elle est située entre les deux électrodes 2 et 3.

Les électrodes 2 et 3 sont constituées par exemple par des métallisations superficielles de feuilles en matière plastique ou encore par des bandes métalliques comme de la feuille d'acier ou de la feuille d'aluminium d'épaisseur de préférence inférieure à 50 microns.

Dans le cas de la feuille sensible chargée (figure 2), l'une 2 de ces électrodes est rapportée sur, ou contre, cette feuille 1 alors que l'autre électrode 3 est maintenue légèrement écartée de ladite feuille 1 par tous moyens désirables et éventuellement rapportée sur une feuille plastique supplémentaire 4..

Dans le cas de la feuille sensible 1 polarisée (figure 4), les deux électrodes 2 et 3 sont rapportées sur, ou contre, respectivement les deux faces de cette feuille 1.

L'ensemble des trois feuilles 1, 2 et 3 est lui-même disposé entre deux feuilles de protection extérieures 5 et 6, dont l'épaisseur est avantageusement comprise entre 0,1 et 0,5 mm, constituées de préférence en une matière élastique, isolante de l'électricité, par exemple du caoutchouc.

Ces feuilles de protection 5 et 6 débordent au-delà de l'ensemble des trois feuilles 1, 2 et 3 et leurs marges débordantes 7 et 8 (figure 1) sont assemblées l'une contre l'autre d'une manière étanche vis-à-vis des impuretés, notamment par collage ou soudage à chaud.

Une pluralité de cales d'écartement 13 sont interposées entre la feuille sensible 1 et l'une, 6, des
feuilles de protection extérieure, les deux faces opposées des cales étant de préférence collées ou soudées
respectivement sur les plages de feuille contre lesquelles elles sont juxtaposées.

Les cales 13 en question peuvent se présenter
sous la forme de grilles ou treillis formés d'éléments
croisés plats réunis de préférence à leurs points de
croisement de façon à ne pas créer de surépaisseurs
locales, ou encore sous la forme de bandelettes plates
parallèles, ou de cales plates espacées, de section
droite carrée, rectangulaire, circulaire ou autre ou
encore sous la forme d'une feuille continue plissée
gaufrée alvéolée ou perforée de part en part ou non.
Dans ce dernier cas, au niveau des bords extérieurs de
l'alèse, les feuilles de protection 5 et 6 débordantes
de l'ensemble des trois feuilles 1, 2 et 3 peuvent
être assemblées par collage ou soudage sur un bord
également débordant de cette feuille continue.

Le matériau constitutif des cales peut être isolant ou conducteur de l'électricité : il est alors
choisi de façon telle que les cales ne risquent pas de
déchirer les feuilles souples contre lesquelles elles
sont placées.

La feuille 1 et la feuille de protection 6 correspondante définissent ainsi entre ces éléments des
chambres ou compartiments 15 ou 16 bien délimités.

L'alèse A formée par la nappe souple composée des
feuilles superposées 1, 2, 3, 5 et 6, présente la
forme générale d'un rectangle dont les côtés mesurent
respectivement entre 10 et 40 cm et entre 20 et 50 cm
s'il s'agit d'une alèse pour nourrissons.

Chacune des électrodes 2 et 3 est reliée à l'aide
de conducteurs 9 et 10 à un ensemble d'exploitation
approprié 11 avantageusement d'un type électronique à
circuits C.MOS propre à exciter un dispositif d'alarme
12 sonore, visuel ou autre.

Pour l'application de l'alèse considérée A à la détection des apnées d'un sujet reposant sur cette alèse, l'excitation de l'alarme 12 est automatiquement assurée lorsque les oscillations, de la tension receuillie entre les électrodes 2 et 3, dues à la respiration normale dudit sujet, s'interrompent pendant une durée supérieure à un seuil prédéterminé, lequel peut être réglé à volonté et être égal par exemple à 10 ou 30 secondes ou présentent une forme s'écartant dans une mesure excessive d'une forme considérée comme normale.

On prévoit en outre, dans la nappe composite, conformément à l'invention une pluralité de surépaisseurs en regard des chambres mais à l'extérieur de ces chambres.

Les surépaisseurs 20 en question peuvent être réalisées en toutes formes et matières désirables.

Elles sont de préférence constituées par des reliefs en saillie sur la face extérieure de l'une au moins des feuilles de protection. Elles peuvent se présenter sous la forme de plots plats 20 (figure 2) ou bombés 21 (figure 4). Pour simplifier la description, ces surépaisseurs en relief seront désignées dans la suite par le mot pastille. Le contour des pastilles est semblable mais plus petit que celui des chambres en regard desquelles elles sont placées de façon à permettre l'enfoncement de la portion de feuille en regard dans les chambres correspondantes lors de l'application d'une pression sur ces pastilles.

Le matériau constitutif des pastilles présente de préférence une dureté et/ou une compressibilité au moins aussi grande que celle du matériau utilisé pour constituer les feuilles de protection de façon que l'application d'une pression sur la pastille se traduise plutôt par une déformation de la feuille de protection concernée que par celle de la pastille elle-même. Ce peut être également le même matériau que celui de ces feuilles.

0143046

Les pastilles peuvent être collées sur les feuilles de protection. Lorsqu'elles sont faites dans le même matériau que ces feuilles, elles peuvent également être venues directement lors de la fabrication de ces feuilles, par moulage ou par calandrage.

Des moyens sont prévus pour éviter des glissements relatifs des diverses couches de l'alèse, glissements qui pourraient se traduire par le déplacement des pastilles par rapport à leur position en regard des chambres. Ces moyens peuvent être constitués par des couches de colle 14 qui maintiennent les feuilles de protection en position bien déterminée par rapport aux cales 13. Ils peuvent également être constitués par des creux ou reliefs complémentaires prévus dans les faces en regard des feuilles, ce qui permet leur accrochage.

Dans chaque cas, la présence des pastilles selon l'invention assure un fonctionnement particulièrement fiable du transducteur et une grande sensibilité.

C'est ainsi que lorsqu'une variation de pression est appliquée au transducteur par l'intermédiaire d'une surface assez grande, par exemple par le dos d'un bébé, la présence des pastilles transmet ces variations de pression préférentiellement au niveau des chambres, ce qui entraîne un certain écrasement de ces chambres.

Dans le premier cas (figures 2 et 3), c'est la distance entre la face chargée $1_1$ et l'électrode 3 en regard qui varie fortement au niveau des différents compartiments 15 délimités entre les cales successives.

Cette disposition est particulièrement avantageuse car le rapprochement de ces éléments sous l'effet de la pression exercée sur la pastille permet de prévoir des cales d'espacement 13 relativement épaisses, par exemple comprises entre 0,5 et 5 mm. Au repos, la capacité électrique de la nappe est alors très faible compte tenu de la grande distance entre électrodes, que cette épaisseur entraîne. Lorsque certaines pastilles sont comprimées, les chambres correspondantes voient, par suite de la grande diminution de distance ci-dessus décrite, leur capacité électrique augmenter fortement, ce qui est très favorable pour l'exploitation des signaux électriques issus des transducteurs.

Dans le second cas (figure 4), les variations de pression exercées localement sur la feuille 1 se traduisent par des enfoncements élastiquement réversibles des portions, de cette feuille, disposées en regard des chambres 16, à l'intérieur de ces chambres, ce qui allonge, et donc amincit ces portions : ce sont surtout ces amincissements réversibles qui sont transformés en variations électriques exploitées aux fins de surveillance ; grâce aux pastilles considérées, lesdites déformations sont accentuées par le fait que les variations de pression sont concentrées dans les régions situées au niveau des chambres.

En se référant à la figure 5, un élément sensible au contact A', de construction générale analogue à celle décrite ci-dessus, et d'aspect incurvé plutôt que plat, est représenté.

Une application possible de cette variante selon l'invention est un assemblage de capteurs sensibles au toucher tels que ceux utilisables sur un bras articulé de robot. Comme il va de soi, les surépaisseurs 20' de l'élément sensible au contact A' améliorent la sensibilité du positionnement du bras articulé de robot.

Par exemple, en prévoyant des connexions électriques individuelles pour identifier de façon unique chacune des surépaisseurs 20', la position de l'assemblage d'éléments sensibles au contact A' par rapport à la surface S peut être obtenue en déterminant quelle surépaisseur 20' est en contact avec cette surface.

En se référant à la figure 6, une variante selon l'invention est illustrée dans laquelle une feuille A" forme un assemblage de capteurs sensibles à la pression analogue à un clavier. La feuille A" est également de constitution analogue à celle discutée précédemment. Chaque surépaisseur 20" correspond à un élément sensible à la pression, les éléments sensibles en question étant arrangés selon des lignes X et des colonnes Y et connectées à des sorties pour fournir un signal d'identification unique à chaque élément sensible enfoncé et pour indiquer de ce fait la position dudit élément sensible.

Dans cette variante, comme dans les autres variantes discutées ci-dessus, l'une au moins des électroaes 2" et 3" peut être divisée en une pluralité d'électrodes séparées et discrètes qui sont associées à chaque surépaisseur 20". Dans cette variante spécifique, montrée sur la figure 6, deux paires d'électroaes supérieures et inférieures, chacune d'entre elles pouvant avoir par exemple la forme d'un rectangle, respectivement $3_1$ et $3_2$ pour les électrodes supérieures et $2_1$ et $2_2$ pour les électrodes inférieures, sont utilisées afin de coopérer pour fournir un signal représentatif de la position pour chaque élément sensible.

Toutes les électrodes $2_1$, associées aux diverses surépaisseurs 20", sont connectées ensembles selon une première direction X, tandis que toutes les électrodes $3_2$ sont connectées selon une seconde direction Y, et les autres électrodes $3_1$ et $2_2$ sont mises à la masse. Dans un tel cas, la pression exercée sur une surépaisseur 20" crée deux signaux électriques qui peuvent être utilisés pour identifier la position de la surépaisseur 20".

0143046

Comme il va de soi, et comme il résulte d'ailleurs déjà de ce qui précède, l'invention ne se limite nullement à ceux de ses modes d'application et de réalisation qui ont été plus spécialement envisagés ; elle en embrasse, au contraire, toutes les variantes.

En particulier, l'une quelconque des deux structures composites décrites ci-dessus pourrait être doublée :

- on pourrait à cet effet faire comprendre en commun l'électrode 3 de la figure 2 par deux structures du type de celle illustrée sur ladite figure 2 et rapporter symétriquement deux ensembles cales-électret-autre électrode-feuille de protection sur respectivement les deux faces de cette électrode 3, cette dernière pouvant être elle-même dédoublée et constituée par métallisation des deux faces de la feuille supplémentaire 4,

- on pourrait également rapporter sur chacune des faces des cales 13 de la figure 4 l'ensemble d'une feuille 1 métallisée sur ses deux faces et revêtue extérieurement d'une nappe de protection. Dans ce cas les cales 13 sont de préférence constitués par une plaque perforée, les régions non perforées de cette plaque jouant le rôle desdites cales.

Dans chacune de ces deux variantes, les pastilles peuvent être situées sur l'une ou sur les deux feuilles de protection extérieure. Egalement, dans chacune de ces deux variantes, les deux électrodes extérieures de la structure "doublée" obtenue peuvent être connectées ensemble et constituer un blindage électrostatique pour la nappe composite.

REVENDICATIONS

1. Nappe composite relativement souple et étendue, constitutive d'un transducteur électromécanique comprenant une feuille sensible à la pression au sens de la transduction électromécanique, feuille interposée entre deux feuilles conductrices ou électrodes elles-mêmes recouvertes extérieurement de feuilles de protection, des cales d'écartement (13) étant interposées entre la feuille sensible (1) et l'une des feuilles de protection (6), cales d'écartement qui délimitent, entre lesdites feuilles, des chambres (15), caractérisée en ce qu'elle présente des surépaisseurs (20) localisées en regard des chambres.

2. Nappe selon la revendication 1, caractérisée en ce que la nappe composite constitue une alèse rectangulaire pour nourrissons dont l'un des côtés est compris entre 10 et 40 cm et l'autre côté entre 20 et 50 cm.

3. Appareil de surveillance des mouvements des êtres vivants, caractérisé en ce qu'il comporte une nappe relativement flexible et étendue, ladite nappe comportant une feuille sensible constituant un transducteur électromécanique produisant un signal électrique en réponse à une pression mécanique, des électrodes situées de part et d'autre de ladite feuille sensible, une feuille de protection placée sur l'une au moins des électrodes, des cales d'espacement interposées entre la feuille sensible et l'une des autres feuilles, afin de définir une pluralité de chambres à l'intérieur de la nappe, et une pluralité de surépaisseurs situées sur la feuille de protection en regard des chambres, et des moyens de surveillance, connectés aux électrodes, propres à contrôler les signaux électriques provenant de la feuille sensible en réponse aux mouvements d'un être vivant en contact avec la nappe, mouvements qui provoquent des variations de pression mécanique sur les surépaisseurs.

4. Assemblage de capteurs sensibles à la pression, caractérisé en ce qu'il comporte une nappe relativement flexible (A'), ladite nappe comportant une feuille sensible constituant un transducteur électromécanique produisant un signal électrique en réponse à une pression mécanique, des électrodes situées de part et d'autre de ladite feuille sensible, une feuille de protection placée sur au moins l'une des électrodes, et des cales d'espacement, interposées entre ladite feuille sensible et l'une desdites autres feuilles, afin de définir une pluralité de chambres à l'intérieur de ladite nappe, ladite nappe incluant des surépaisseurs (20') situées en regard desdites chambres et définissant les éléments sensibles des capteurs dudit assemblage.

5. Assemblage de capteurs sensibles à la pression selon la revendication 4, caractérisé en ce qu'une pluralité d'électrodes sont présentes sur les deux faces de la feuille sensible, lesdites électrodes fournissant à des moyens d'exploitation des signaux électriques représentatifs de celles, des surépaisseurs (20"), qui sont soumises à une variation de pression.

6. Assemblage de capteurs sensibles à la pression selon la revendication 5, caractérisé en ce que chaque surépaisseur (20") est associée à deux paires d'électrodes, savoir deux inférieures ($2_1$, $2_2$) et deux supérieures ($3_1$, $3_2$), ces électrodes étant arrangées de telle sorte que les premières électrodes ($2_1$ et $3_1$) de chaque paire soient situées en regard l'une de l'autre, que les secondes électrodes ($2_2$ et $3_2$) de chaque paire soient situées en regard l'une de l'autre et que toutes les premières électrodes inférieures ($2_1$) associées aux diverses surépaisseurs (20") soient connectées entre elles selon une première direction X, que toutes les secondes électrodes supérieures ($3_2$) soient connectées entre elles selon une seconde direction Y, et que les autres électrodes ($3_1$ et $2_2$) soient mises à la masse.

7. Structure composite selon l'une quelconque des précédentes revendications, caractérisée en ce que les surépaisseurs sont constituées par des reliefs faisant saillie sur la face extérieure de l'une au moins des feuilles de protection.

8. Structure composite selon l'une quelconque des précédentes revendications, caractérisée en ce que les surépaisseurs ont un contour semblable à celui des chambres en regard desquelles elles sont placées mais plus petit.

9. Structure composite selon l'une quelconque des précédentes revendications, caractérisée en ce que les surépaisseurs ont une épaisseur non uniforme, décroissante vers la périphérie.

10. Structure composite selon l'une quelconque des précédentes revendications, caractérisée en ce que les surépaisseurs sont constituées en un matériau de dureté au moins égale à celles des feuilles de protection.

11. Structure composite selon l'une quelconque des revendications 1 à 9, caractérisée en ce que les surépaisseurs sont constituées en un matériau de compressibilité au moins égale à celle des feuilles de protection.

12. Structure composite selon l'une quelconque des précédentes revendications, caractérisée en ce que les surépaisseurs font partie intégrante des feuilles de protection.

13. Structure composite selon l'une quelconque des précédentes revendications, caractérisée en ce que les surépaisseurs ont une épaisseur comprise entre 0,1 et 5mm.

14. Structure composite selon l'une quelconque des précédentes revendications, caractérisée en ce que les diverses couches de la nappe comprennent des moyens pour empêcher le déplacement au cours du temps des surépaisseurs par rapport aux chambres.

15. Structure composite selon l'une quelconque des précédentes revendications, caractérisée en ce que la feuille sensible comporte un film piézoélectrique.

FIG.1.

0143046

Fig.2.

Fig.3.

Fig.4.

FIG.5.

FIG.6.